# EUROPEAN PATENT SPECIFICATION

(11) **EP 1 224 600 B1**
(45) Date of publication and mention of the grant of the patent: **13.06.2007**
(21) Application number: 00967752.7
(22) Date of filing: 22.09.2000
(51) Int. Cl.: G06F 19/00, G06F 1/00

(54) **PATIENT DATA MONITORING SYSTEM**
SYSTEM ZUM ÜBERWACHEN VON PATIENTENDATEN
SYSTEME DE SURVEILLANCE DE DONNEES DE PATIENT

(30) Priority: 01.10.1999 GB 9923273; 09.05.2000 GB 0011029; 22.08.2000 GB 0020541
(43) Date of publication of application: 24.07.2002
(73) Proprietor: GLAXO GROUP LIMITED, Greenford, Middlesex UB6 ONN (GB)
(72) Inventor: ANDERSON, Gregor John McLennan, Glaxo Wellcome plc, Ware, Herts. SG12 0DP (GB); BONNEY, Stanley George, Glaxo Wellcome plc, Ware, Herts. SG12 0DP (GB); JONES, Anthony Patrick, Glaxo Wellcome plc, Ware, Herts. SG12 0DP (GB); ROBERTSON, Duncan, Glaxo Wellcome plc, Ware, Herts. SG12 0DP (GB)
(74) Representative: Pike, Christopher Gerard
(86) International application number: PCT/EP2000/009292
(87) International publication number: WO 2001/026020

(56) References cited:
- EP-A- 0 884 670
- WO-A-99/41682
- US-A- 5 338 157
- US-A- 5 924 074

## Description

The present invention relates to a system for the remote monitoring of the medical condition of plural patients. The system employs plural patient electronic data collectors, each for collecting patient data relevant to a particular patient's medical condition. Each patient electronic data collector is capable of communicating with an entrypoint to a network computer system.

It is common prescribing practice for a doctor to prescribe a patient with medicament in a medicament dispenser together with instructions for patient administration of the medicament according to a defined treatment regime. The patient typically therefore, receives instructions relating to the correct use of the dispenser together with recommended dosing amounts, dose intervals and treatment period. The patient is then trusted to follow the treatment regime as set by the doctor.

A limitation associated with this practice is that the treatment regime is set at the time of prescription and cannot therefore account for changes in the patient's condition over the treatment period. A further limitation associated with this practice is that the onus is on the patient to comply with the doctor's instructions. Occasionally, patients will forget to take the medicament or will vary the treatment regime in an unpredictable manner with possible consequences for the success of the treatment.

A variation on the above-described prescribing practice involves the use by a patient of a diagnostic device which enables data relating to their medical condition to be gathered on a regular basis. This data may for example, be collected prior to administration of any medicament and a correct dose amount calculated on the basis of the diagnostic data. An example of this practice would be that of a diabetic who checks their blood-sugar levels in order to calculate a required dose of insulin.

In developments of the practice variation, the diagnostic device may be integrated with the delivery system. Information relating to the patient's condition and usage of the dispenser may thus be displayed to the patient to enable the better management of their medical condition. The information may further be stored in a memory such that it may be recalled at a later time to enable historic analysis of the progress of the condition and effect of the treatment. Dispensers employing electronic data management systems have been proposed for this purpose.

It is desirable to have a system which allows for the remote monitoring of the medical condition of plural patients having similar medical conditions and undergoing similar treatment or prescribing regimes. This monitoring would allow for an overview picture to be obtained of the degree and nature of patient compliance with the treatment or prescribing regime. The effectiveness of the treatment or prescribing regime in the group of patients may also be monitored over time. Where different sets of plural patients are undertaking different treatment or prescribing regimes the method would further enable cross-comparison of the effectiveness of the different regimes. It is further desirable that the system allows for selective access to the collected data. For example, the patient and their doctor may be allowed exclusive access to the individual patient data. Healthcare managers on the other hand may be enabled to view overview data, but not data relating to individuals.

The Applicants have now developed an improved system for remotely collecting and providing selective access to medical data relevant to plural patients having related medical conditions. The system employs plural electronic data collectors, each of which is assigned to a particular patient. Associated with each electronic data collector there is provided a communicator for communicating with an entrypoint to a remote network computer system to enable transfer of patient data thereto. The data is transferable to a first database which is specific to the patient and to a second database which is not patient-specific but is specific to the medical condition being monitored. The system enables different levels of security to be applied to access to each database. First and second secure access gateways are provided permitting access to the first and second databases in response to first and second user authorisation commands.

The system is configurable to allow selective access to the different databases depending upon the level of the user authorisation. The system can further be integrated with other medical information databases including medicament databases or systems for the provision of electronic prescriptions. In the latter case, the systems in combination enable seamless remote assessment or medical condition and prescription of medicament or variation of the prescription regime.

US-A-5,363,842 describes an inhalation device for use in delivering inhalable medicament. The device enables data relating to the patient's breathing pattern to be collected, analysed and displayed to the patient. The data is stored in a memory for download to a workstation at the clinic.

WO99/35588 describes a method for managing the administration of medicine and in particular, monitoring patient compliance with a prescribed treatment regime. The method relies on input of patient data to a central computer workstation. The central computer workstation calculates and transmits dosage data to a dispensing device via a communications link. The dispensing device delivers drug in accord with the dosage data.

US-A-5,924,074 describes a patient electronic medical records system in which access to patient records may be provided to different authorized users on a preferential basis.

EP-A-884670 A1 describes a secure database for comprising medical data.

According to one aspect of the present invention there is provided a system for collecting and providing selective access to medical data relevant to plural patients having related medical conditions comprising a network computer system; associated with the network computer system, a first patient-specific database and a second condition-specific database; remote from said network computer system, plural patient electronic data collectors, each for collecting patient data relevant to a particular patient's medical condition; associated with each patient electronic data collector, a communicator for communicating with an entrypoint to said network computer system to enable transfer of said patient data to said first patient-specific database and to said second condition-specific database; a first secure access gateway permitting access to the first patient-specific database in response to a first user authorisation command; and a second secure access gateway permitting access to the second condition-specific database in response to a second user authorisation command, wherein said first patient-specific database is set up to store the patient data relevant to plural particular patients arranged on a patient-specific basis, and said second medical condition-specific database is set up to store the patient data relevant to plural particular patients arranged on a medical condition-specific basis.

In one aspect, the patient electronic data collector collects patient data on a regular basis. In another aspect, the patient electronic data collector collects patient data on a continuous basis.

The patient data is relevant to the patient's medical condition. The data may therefore comprise diagnostic data which is of use in diagnosing the patient's ongoing condition. The data may also comprise compliance data which is of use in assessing a patient's compliance with a particular treatment or prescription regime. It will be appreciated, particularly in the light of the further description presented below, that in aspects herein the patient data may be selectively transferred to the first and second database such that not all patient data is necessarily transferred to both databases.

The system is of most application where the plural patients have related medical conditions. This is also likely to mean that similar or at least related diagnostic data will be collected from each patient. The patients may also be on similar, related or identical treatment or prescribing regimes. Cross-comparison of the effectiveness of different treatment or prescribing regimes may be undertaken. The system may also be employed in a clinical trial situation wherein the prescription regime for at least some of the patients involves the use of a placebo.

The patient electronic data collectors are physically, and potentially geographically distant from the entrypoint to the network computer system. It is envisaged that the each electronic data collector will be kept under the control of an individual patient. In embodiments, the data collector may be worn by the patient or be a handheld device carried by the patient. Examples of patient-wearable devices would include belt attachable devices, devices in the form of watches for wrist or leg attachment and devices attachable as jewellery. Suitable body attachment means will be incorporated as required.

Any patient electronic data collector may in a preferred aspect be integrated with a system for the delivery of medicament. The medicament delivery system will typically comprise a medicament container and a dispensing mechanism for dispensing medicament from the medicament container. The system may be arranged to collect data when the patient uses the medicament delivery system.

In one aspect, the medicament delivery system provides respirable delivery of medicament to the patient. In another aspect, the medicament delivery system provides injectable delivery of medicament to the patient.

In one aspect the medicament delivery system and electronic data collector are comprised within a handheld device. In another aspect, the medicament delivery system is an implant in the body of the patient.

Suitably, the medicament delivery system includes a predictive algorithm or look-up table for calculating the optimum amount of medicament to dispense. Suitably, the medicament delivery system has a memory including a dose memory for storing dosage data and reference is made to the dose memory in calculating the optimum amount of medicament to dispense.

Suitably, the system additionally comprises a selector for selecting the amount of medicament to dispense from a medicament dispensing mechanism. The selector may thus be employed to vary the medicament dose for dispensing from the dispensing mechanism.

In one aspect, the selector is manually operable.

In another aspect, the selector is operable in response to a signal from the transmitter.

Suitably, the selector comprises a timing mechanism for varying the time interval of actuation of the dispensing mechanism.

Alternatively, the selector comprises a metering mechanism between the medicament container and the dispensing mechanism for metering a variable quantity of medicament for dispensing.

Alternatively, the selector comprises a multiple-fire mechanism for multiple actuation of the dispensing mechanism, wherein each actuation releases a portion of the optimum amount of medicament. Successive actuations may be pulsed, for example such that the time intervals between actuations may be based on arithmetic or geometric progressions.

Suitably, the system additionally comprises a detector for detecting dispensing from the medicament container, wherein said detector communicates dispensing data to the electronic data management system.

Suitably, the communicator is local to the patient, for example being integral with the patient data collector, or within another handheld device or present in the home or working environment of the patient. The communicator may for example, be comprised within a device which is mechanically coupled to the patient data collector by any suitable mechanical mechanism including grip mechanisms and snap-fit mechanisms. In a preferred aspect, the data communicator forms a snap-in module and the patient data collector is shaped for receipt of the module.

In one preferred aspect, the communicator communicates wirelessly with the entrypoint to the network computer system.

The patient data typically includes a patient identifier. The identifier may be freely readable or it may be masked to ensure patient anonymity.

Suitably, the data is communicable between the patient electronic data collector and the network computer system in encrypted form. All suitable methods of encryption or partial encryption are envisaged. Password protection may also be employed.

In one aspect, the patient data is continuously communicable between the patient electronic data collector and the network computer system. In another aspect, the patient data is communicable in packet form between the patient electronic data collector and the network computer system.

The network computer system may in aspects comprise a single server or it may comprise plural servers arranged for communication with each other. In one aspect, the network computer system comprises a public access network computer system. The Internet is one suitable example of a public access network computer system, wherein the entrypoint can be any suitable entrypoint thereto including gateways managed by an Internet service provider. The public access network computer system may also form part of a telecommunications system, which may itself be either a traditional copper wire system, a cellular system or an optical or microwave network.

In another aspect, the network computer system comprises a private access network computer system and the entrypoint is a secure gateway. The private access network system may for example, comprise an Intranet or Extranet which is maintained by a private organisation.

The network computer system is typically located at or under the control of a specialist healthcare data manager. The data manager may for example be associated with a healthcare provider or manager such as a doctor's practice, a hospital, a healthcare management centre or a pharmaceutical company. It is an advantage of the system herein, that the network computer system may be located geographically distant from the patient.

The first database comprises medical condition data which is specific to a particular patient. It will thus be appreciated that over a period of time various sets of data will be transferred from the patient electronic data collector to the first database. The first database may therefore be used to track the condition of the patient and their compliance with any treatment regime over a period of time.

The second database is a collective database comprising medical condition data which relates to plural patients. It will thus be appreciated that over a period of time various sets of data relating to each of the plural patients will be transferred from each of the patient electronic data collectors to the second database. The data may be masked such that it is not directly relatable to any particular patient source. The second database may therefore be used to track the condition of the plural patients and their compliance with any treatment regime over the period of time.

The first and second databases may be arranged to be entirely separate from each other. Alternatively, the first database specific to each patient may be arranged to comprise a sub-database of the larger second database. Hybrids of these two alternatives are also envisaged.

The data within any of the databases may be partitioned in any suitable way. The partitioning may for example, reflect a patient's request that certain information is kept confidential whereas other information is made more generally available. Segments of any database may be arranged to require special security access or indeed be maskable. The masking may occur at the time of data collection such that only certain of the collected data are transferred to any of the databases. The partitioning may also be arranged for commercial reasons. Commercial partitioning may for example be arranged to reflect a pricing structure for access to the databases, or to protect commercial confidentiality.

Access to the databases herein will typically be achieved by the user initially accessing a web-site from which the databases may be accessed through the secure gateways. The initial web-site may be a general public access site, but more usefully the site may be tailored to meet the user's needs. For example, if the user is a patient the initial web-site may comprise links to other sites of interest to the patient or to patient chat rooms. Alternatively, if the user is a healthcare professional links may be provided to the sites of professional interest groups.

The first secure access gateway permits access to the first patient-specific database in response to a first user authorisation command. The second secure access gateway permits access to the second condition-specific database in response to a second user authorisation command. The first and second secure access gateways may be arranged to be entirely distinct from each other. Alternatively, the first and second secure access gateways may be coupled or arranged in a series fashion. A further secure access gateway may additionally be provided through which access a user must first gain access in order to gain access to the first and second secure access gateways.

The secure gateways will typically provide password protection and the different user authorisation commands will take the form of different passwords. The gateways or any other part of the system may also incorporate a firewall or suitable encryption means.

The first and second user authorisation commands will usually be distinct, but embodiments can be envisaged when a single authorisation command gains access through both gateways.

Suitably, the authorised users are selected from the group consisting of the patient, a healthcare professional such as a doctor or nurse, a pharmacist, an emergency assistance provider, a research professional, a database manager and any combinations thereof.

Suitably, the first and second secure access gateways allow for different levels of access authorisation to the first and second databases to be assigned to different authorised users.

The users that are provided access rights to the first and second databases will probably be different but members of the user groups may overlap. The first database clearly contains information which may be sensitive to the patient and user access may thus be restricted to just the patient and their doctor. The second database contains information which may provide an overview of the effectiveness of a particular treatment regime. This information may be medically, or even commercially, sensitive and user access may be accordingly restricted.

Embodiments are envisaged in which access to either one or both of the databases involves payment of a fee or is rewarded by an incentive. For example, to encourage participation in the data collection the patient may receive a small payment each time data is communicated to the first database. Alternatively, a commercial access fee may be charged for access to the second database and that fee may be varied depending on the nature, volume and commercial value of the data accessed. Any fees or incentives are deductible or awardable by electronic means.

The user authorisation commands may be issued to groups of users having shared interests of forming alliances of interest groups. Patient groups may thus be given group access rights or similarly practitioner groups may be given group user authorisations. Any suitable groupings or alliances are envisaged arranged along any suitable lines including patient groups, professional groups or commercial alliances.

The system is arranged to allow selective monitoring and access to data. A key part of the system is that it enables patient preferences to be reflected via the use of patient permissions and authorisations. Permission steps may therefore be introduced at the time of data collection, data transfer, data storage and data access. The permissions may be arranged to be alterable on request of the patient. The system may also be configured to accommodate situations where the patient waives their right to control of the collected data by contractual arrangement with the administrators of the system.

Suitably, information from a remote datasource (i.e remote from the network and/or remote from the patient) is made available to the user at the initial point of access to the network computer system. The information may be historic or it may be updated regularly or even be provided in real-time.

In one aspect, the remote datasource comprises data relating to ambient environmental conditions such as weather conditions, or pollution, smog and pollen levels.

In another aspect, the remote datasource comprises a database of prescribable medicaments. The database may be arranged to focus on medicaments of particular applicability to the patient's condition and to be the subject of regular update as new medicaments are released to the market.

The remote datasource may, for example be managed by a medicament prescriber, for example a doctor's practice. Information transferred from the medicament prescriber may thus, comprise changes to prescription details, automatic prescription updates or training information.

In another aspect, the remote datasource is managed by a pharmacy information transferred from the pharmacy may thus, comprise information relating to the medicament product.

In a further aspect, the remote datasource is a manufacturer of medicament or medicament delivery systems. Information transferred to the system may thus, comprise product update information.

In a further aspect, the remote datasource is a research establishment. In a clinical trial situation, information may thus be transferred relating to the trial protocol.

Suitably, the patient electronic data collector further comprises a patient electronic data management system comprising a memory for storage of data; a microprocessor for performing operations on said data; and a transmitter for transmitting a signal relating to the data or the outcome of an operation on the data. The memory may comprise a non-volatile memory chip (e.g. an EEPROM or a FLASH memory chip) which is capable of storing data when the electronic data collector is turned off.

Suitably, the patient electronic data management system additionally comprises a geographic positioning system such as a global positioning system or a system which relies on the use of multiple communications signals and a triangulation algorithm.

Either the network computer system or the communicator may initiate data transfer. Suitably, the communicator enables two-way transfer of data between the network computer system and the patient electronic data management system.

Suitably, the system additionally comprises an authorised user (i.e. other than the patient) data communicator comprising an authorised user electronic data management system comprising a memory for storage of data; a microprocessor for performing operations on said data; and a transmitter for transmitting a signal relating to the data or the outcome of an operation on the data; and a communicator for communicating, preferably wirelessly, with an entrypoint to a network computer system to enable communication of data between the network computer system and the authorised user electronic data management system.

The system herein may be integrated with a system for electronic prescribing. Thus, according to another aspect of the present invention there is provided a system for the remote assessment of a patient medical conditions and remote prescription therefor comprising a system as described above and additionally a first authorised user (e.g. a prescriber) data communicator capable of communicating a prescription authorisation command to the network computer system; and a second authorised user (e.g. a pharmacist) data communicator capable of receiving a prescription authorisation command from the network computer system.

Suitably, any communicator employs radiofrequency or optical (e.g. infra red or ultraviolet) signals.

In one aspect, any communicator communicates with the network computer system via a gateway thereto. Communication will typically involve the use of a suitable communications protocol.

In another aspect, the system (device) is provided with an embedded network server to enable it to be comprised directly within the network system, typically using IP protocol. The embedded network server will have hardware and software components and for example comprise an HTTP (web) server, an FTP (file) server or an SMTP (mail) server. The embedded network server will typically be provided with a unique network address such as a web-site address, an e-mail address or a file transfer protocol address. The so-enabled system (device) may also have the capability to form local area networks with other similarly enabled systems (devices) to enable local transfer of data therebetween.

Suitably, the communicator communicates with network computer system via a second communications device. The second communications device may itself comprise an embedded web server. Preferably, the second communications device is a telecommunications device, more preferably a cellular phone or pager. Preferably, the communicator communicates with the second communications device using spread spectrum radiofrequency signals. A suitable spread spectrum protocol is the Bluetooth (trade mark) standard which employs rapid (e.g. 1600 times a second) hopping between plural frequencies (e.g. 79 different frequencies). The protocol may further employ multiple sending of data bits (e.g. sending in triplicate) to reduce the effect of interference.

In another aspect, the communicator communicates with the second communications device using an infra red data communications standard (e.g. IrDA).

In one aspect, the network computer system comprises a public access network computer system. The Internet is one suitable example of a public access network computer system, wherein the entrypoint thereto is typically managed by an Internet service provider. The public access network computer system may also form part of a telecommunications system, which may itself be either a traditional copper wire system, a cellular system or an optical or microwave network.

In another aspect, the network computer system comprises a private access network computer system typically comprising a private entrypoint system. The private access network system may for example, comprise an intranet or extranet which may for example, be maintained by a health service provider or medicament manufacturer. The private access network system may for example include password protection; a firewall; and suitable encryption means.

Suitably, the patient electronic data management system additionally comprises a datalink for linking to a local data store such as a personal computer or set-top box to enable communication of data between the local data store and the microprocessor. Preferably, the datalink comprises an infrared emitter and sensor.

Suitably, the patient electronic data management system additionally comprises a data input system for user input of data to the electronic data management system. More preferably, the data input system comprises a man machine interface preferably selected from a keypad, voice recognition interface, graphical user interface (GUI) or biometrics interface.

Suitably, the patient electronic data management system additionally comprises a display for display of data from the electronic data management system to the user. The display may for example, comprise a screen such as an LED or LCD screen.

In one aspect, the system is suitable for the remote assessment of plural patients' respiratory conditions and each patient electronic data collector additionally comprises a sensor which senses the breath of a user, wherein the sensor communicates breath data to the patient electronic data collector.

Suitably, the sensor comprises a breath-movable element which is movable in response to the breath of a patient. Preferably, the breath-movable element is selected from the group consisting of a vane, a sail, a piston and an impeller.

In another aspect, the sensor comprises a pressure sensor for sensing the pressure profile associated with the breath of a user.

In a further aspect, the sensor comprises an airflow sensor for sensing the airflow profile associated with the breath of a user.

In a further aspect, the sensor comprises a temperature sensor for sensing the temperature profile associated with the breath of a user. The temperature of the inhaled and exhaled part of the breath cycle varies and may, thus, be used as a measurement tool.

In a further aspect, the sensor comprises a moisture sensor for sensing the moisture profile associated with the breath of a user. The moisture content of the inhaled and exhaled part of the breath cycle varies and this also may be used as a measurement tool.

In a further aspect, the sensor comprises a gas sensor for sensing the oxygen or carbon dioxide profile associated with the breath of a user. The chemical profile of the inhaled and exhaled part of the breath cycle varies and this further may be used as a measurement tool.

Suitably, the breath data includes breath cycle data or peak flow data.

In one aspect, the system is suitable for the delivery of respirable medicament and additionally comprises a sensor which senses the breath of a user, wherein the sensor communicates breath data to the electronic data management system.

Suitably, the system additionally comprises an actuator for actuating the dispensing mechanism, said actuator being actuable in response to a trigger signal from the transmitter.

Suitably, the electronic data management system includes a predictive algorithm or look-up table for deriving from the breath data when to transmit the trigger signal. For example, a real-time analysis of the patient breath waveform may be made and the trigger point derived by reference to that analysed waveform.

Suitably, the memory includes a dose memory for storing dosage data and reference is made to the dose memory in calculating the optimum amount of medicament to dispense.

Suitably, the system additionally comprises a detector for detecting dispensing from the medicament container, wherein said detector communicates dispensing data to the patient electronic data management system.

In one preferred aspect, the medicament container is an aerosol container and the dispensing mechanism is an aerosol valve.

In another preferred aspect, the medicament container is a dry-powder container, that is to say a container suitable for containing medicament in dry-powder form.

Suitably, the actuator comprises an energy store for storing energy which energy is releasable to actuate the dispensing mechanism of the medicament container. The energy store comprises in preferred aspects, a biasable resilient member such as a spring, a source of compressed fluid such as a canister of compressed gas or a battery. Chemical energy sources are also suitable and might include chemical propellant or ignition mixtures. Other sources might include physical explosives such as liquefied or solidified gas in a canister which burst when heated or exposed to the atmosphere.

The system may additionally comprise a safety mechanism to prevent unintended multiple actuations of the actuator. The patient is thereby protected from inadvertently receiving multiple doses of medicament in a situation where they take a number of short rapid breaths. More preferably, the safety mechanism imposes a time delay between successive actuations of the actuator. The time delay is typically of the order of from three to thirty seconds.

An actuation counter which can be mechanical or electronic may be provided to the system.

A medicament dispensing counter, such as a dose counter, may be provided to the system. This may be mechanical or electronic. The counter may be coupled to a visual display to provide feedback to the patient as to amount of drug released or remaining in the container.

A manual override can be provided to the system for use in the event of emergency or system failure.

In another aspect, the system is suitable for the remote assessment of a plural patient's cardiovascular condition and each patient electronic data collector additionally comprises a sensor which senses the cardiovascular activity of a patient, wherein the sensor communicates cardiovascular data to the patient electronic data collector. Preferably, the sensor measures the blood pressure of the patient.

According to another aspect of the present invention there is provided a method for collecting and providing selective user access to medical data relevant to plural patients having related medical conditions comprising locally collecting patient data relevant to each patient's medical condition in electronic form; communicating with an entrypoint to said network computer system to enable transfer of said patient data to a first patient-specific database and to a second condition-specific database of said network computer system; and permitting first authorised user access to the first patient-specific database via a first secure access gateway, and/or permitting second authorised user access to the second condition-specific database via a second secure access gateway, wherein said first patient-specific database is set up to store the patient data relevant to plural particular patients arranged on a patient-specific basis, and said second medical condition-specific database is set up to store the patient data relevant to plural particular patients arranged on a medical condition-specific basis.

Suitably, the method comprises collecting the data on a regular basis.

Suitably, the method comprises collecting the data on a continuous basis.

Suitably, the method comprises communicating, preferably wirelessly, the data in encrypted form.

In one aspect, the data is continuously communicable. In another aspect, the data is communicable in packet form.

Suitably, the method comprises permitting different levels of access to the data to different authorised users. In one aspect the method is suitable for remotely assessing a patient's condition and remotely prescribing therefor and additionally comprises a first authorised user communicating a prescription authorisation command to the network computer system; a second authorised user receiving said prescription authorisation command from the network computer system; and said second authorised user preparing the prescription based on the prescription authorisation.

In another aspect, the method is suitable for remotely assessing a patient's condition and remotely prescribing therefor and additionally comprises a first authorised user (e.g. a doctor) communicating a prescription authorisation command to a pharmacy network computer system; a second authorised user (e.g. a pharmacist) receiving said prescription authorisation command from the pharmacy network computer system; and said second authorised user preparing the prescription for the patient based on the prescription authorisation. The pharmacy network computer system is arranged for communication with the network computer system.

Suitably, the first authorised user communicates the prescription authorisation in response to a 'update prescription' alerting signal visible at a patient-specific network address. The 'update prescription' signal is typically based on data communicated from the patient data collector which may for example, reflect a change in the patient's condition. In one aspect, where the patient data collector forms part of a medicament delivery system the 'update prescription' signal may be an alert that the levels of medicament in the delivery system are running low and that a re-prescription is needed.

According to a further aspect of the present invention there is provided a network computer system for use with the remote assessment system described above comprising an interface capable of receiving patient data in electronic form; associated with said interface, a first patient-specific database and a second condition-specific database for storing said patient data; and an authorised user inquiry system comprising either or both of
(a) a first secure access gateway permitting access to the first patient-specific database in response to a first user authorisation command; first search means for searching said patient-specific database in response to a first authorised user inquiry; and first results transmitting means for transmitting the results of said first authorised user inquiry to the first authorised user;
(b) a second secure access gateway permitting access to the second condition-specific database in response to a second user authorisation command; second search means for searching said second condition-specific database in response to a second authorised user inquiry; and second results transmitting means for transmitting the results of said second authorised user inquiry to the second authorised user, wherein the patient data originates remotely from the network computer system, and wherein said first patient-specific database is set up to store the patient data relevant to plural particular patients arranged on a patient-specific basis, and said second medical condition-specific database is set up to store the patient data relevant to plural particular patients arranged on a medical condition-specific basis.

Suitably, the data is received at a patient-specific network address, which may be permanently assigned or dynamically assigned on a temporary basis. The network address comprises a web-site address or a file transfer protocol address or other suitable web-hub on a public access network computer system such as the Internet, or a private access network computer system such as an Extranet or Intranet. The patient data arises from a patient data collector local to the patient and in wireless communication with an entrypoint to the network computer system. The authorised user inquiry originates from any suitable authorised user. The system may be configured to allow for selective access to the patient database dependent upon level of user authorisation.

According to a further aspect of the present invention, there is provided a method for collecting and providing selective user access to medical data relevant to plural patients having related medical conditions, the method comprising receiving data relevant to each patient's medical condition collected automatically at a plurality of locations; storing some or all of the received data in a first, patient-specific database; storing some or all of the received data in a second, condition-specific database; allowing access to said first database in response to a first user authorisation command; and allowing access to said second database in response to a second user authorisation command, wherein said first patient-specific database is set up to store the patient data relevant to plural particular patients arranged on a patient-specific basis, and said second medical condition-specific database is set up to store the patient data relevant to plural particular patients arranged on a medical condition-specific basis.

According to a further aspect of the present invention, the method described above may be implemented in the form of computer software. The software may comprise a computer program comprising program code means for, when executed on a computer, instructing a computer to perform all of the steps of the method. The software may also comprise a computer program product comprising a computer readable recording medium having recorded thereon a computer program comprising code means for, when executed on a computer, instructing said computer to perform the steps of the method.

Embodiments of systems according to the invention will now be described with reference to the accompanying drawings in which:
Figure 1. is a schematic representation of a first system in accord with the present invention in which a patient electronic data collector forms part of a medicament delivery system;
Figure 2. is a schematic representation of a second system in accord with the present invention in which a patient electronic data collector forms part of a medicament delivery system;
Figures 3 and 4 are schematic representations of third and fourth systems in accord with the present invention in which the patient remote assessment system integrates with a system for electronic prescription of medicament;
Figure 5. is a system diagram of a third system in accord with the present invention;
Figure 6. is a system diagram of a central controller unit for use in accord with the present invention;
Figures 7. and 8. are schematic diagrams of first and second database structures for use in accord with the present invention;
Figure 9. is a system diagram of a patient electronic data manager for use in accord with the present invention;
Figure 10. is a flow diagram illustrating the steps of data collection and data communication to the network in accord with the present invention; and

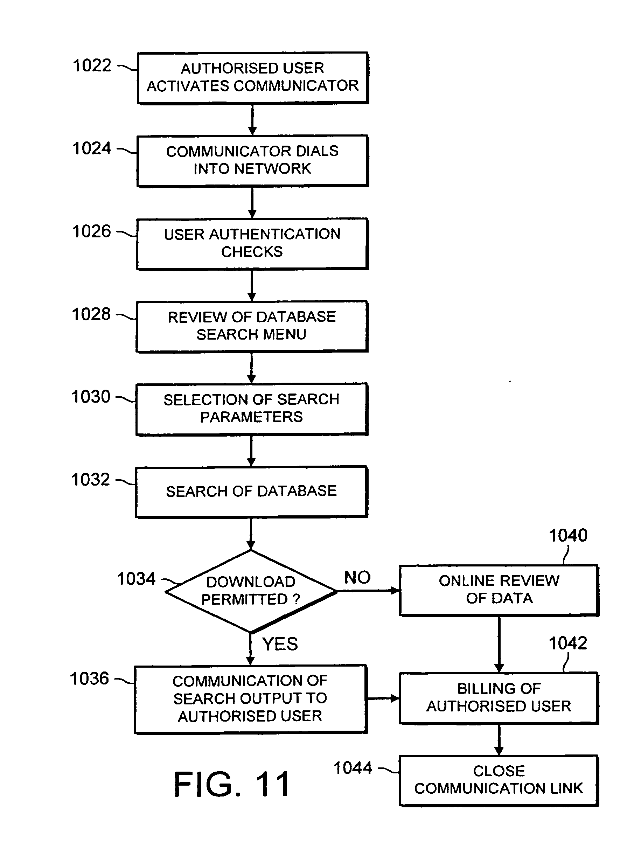

Figure 11. is a flow diagram illustrating the steps of data access at the network in accord with the present invention.

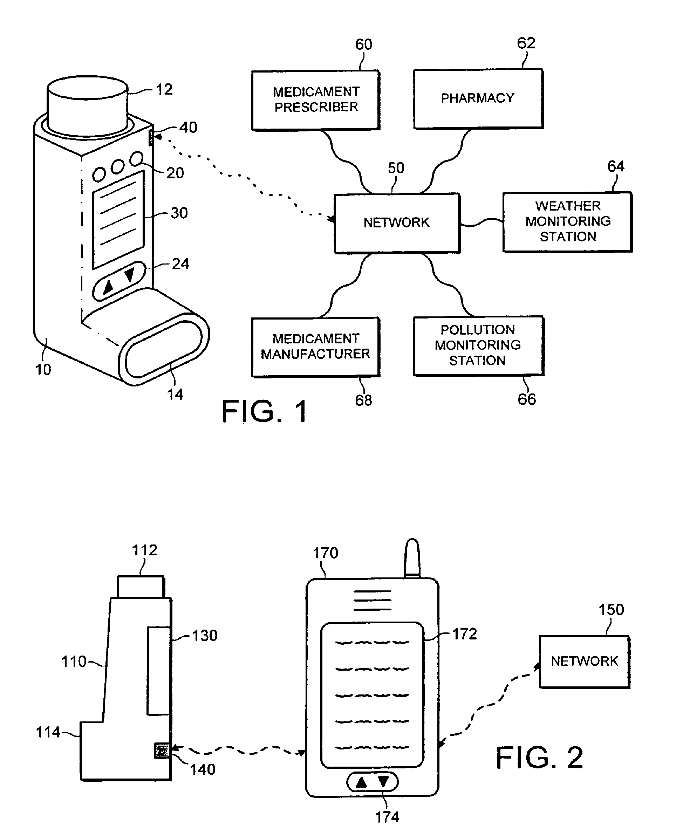

Figures 1 and 2 show representative parts of systems herein in which the patient electronic data collector is comprised within a metered dose inhaler 10 (or 110) for the delivery of inhalable medicament. The inhaler 10 (or 110) is provided with communication means 40 (or 140) to enable communication with a network computer system 50 (or 150). It will be appreciated that the full system will comprise plural such patient electronic data collectors. It will also be appreciated that the network computer system will have a defined data structure as will become apparent when reference is made to later Figures presented herein.

Referring to Figure 1 in more detail, the inhaler comprises a tubular housing 10 in which an aerosol container 12 is located. The housing is open at one end (which will hereinafter be considered to be the top of the device for convenience of description) and is closed at the other. A dispensing outlet 14 leads laterally from the closed end of the housing 10. In the embodiment illustrated, the outlet 14 is in the form of a mouthpiece intended for insertion into the mouth of the patient but it may, if desired, be designed as a nozzle for insertion into the patient's nostril.

The aerosol container 12 is located in the housing 10 so that one end protrudes from the open top of the housing 10. The aerosol container 12 has an outlet valve stem (not visible) at one end which connects with a support (not shown) in the housing 10. To dispense the dose, the protruding portion of the aerosol container 12 is depressed to move the container 12 relative to the valve stem to open the valve and dispense medicament into the outlet 14 from which it can be inhaled by a patient.

The dispenser includes an electronic data management system in the form of an integrated circuit preferably integrated into one or more integrated circuit chips and comprised within the housing (not visible). The user may access the electronic data management system by use of push-buttons 20 and toggle menu-button 24. Display 30 allows for display of menu choices and data from the electronic data management system. The dispenser communicates via communications transceiver 40 to network computer system 50. The network computer system 50 comprises a secure network computer system which will typically be under the control of a healthcare data manager. Remote information sources 60, 62, 64, 66, 68 also have access to the network. In more detail, the remote information sources comprise a medicament prescriber 60, a pharmacy 62, a weather monitoring station 64, a pollution monitoring station 66 and a medicament manufacturer 68. Two-way data transfer is possible between the electronic data management system and the network computer system 50 via the communications transceiver 40. Information transfer is thus possible between the electronic data management system and any of the remote information sources 60, 62, 64, 66, 68. Information received from any of the remote information sources 60, 62, 64, 66, 68 may be utilised by the electronic data management system to vary the recommended medicament dose for delivery to the patient.

Figure 2. shows a variation of the system of Figure 1. The system comprises standard-form metered dose inhaler for the delivery of inhalable medicament comprising tubular housing 110, an aerosol container 112 and dispensing outlet 114. Operation of the inhaler is as described above with reference to Figure 1.

The dispenser includes an electronic data management and communications system 140 comprised within the housing 110. Display 130 allows for limited display data from the electronic data management system. The dispenser readily communicates via communications system 140 to palmtop computer 170. The communication is via spread spectrum radiofrequency signals operable over a relatively short range (e.g. up to ten metres). The palmtop computer 170 has a more sophisticated display 172 including a graphical user interface comprising menu-entry screens from which selections may be made using toggle menu-button 174.

The patient accesses the electronic data management system 140 of the dispenser through the palmtop computer 170. The palmtop computer 170 itself can communicate through a telecommunications link with network computer system 150. The network computer system 150 comprises a secure Extranet computer system. As in Figure 1, remote information sources may also have access to the Extranet. Two-way data transfer is possible between the electronic data management system and the network computer system 150 via the communications links with the palmtop computer 170. Information transfer is thus possible between the electronic data management system 140, palmtop computer 170 and any of the remote information sources.

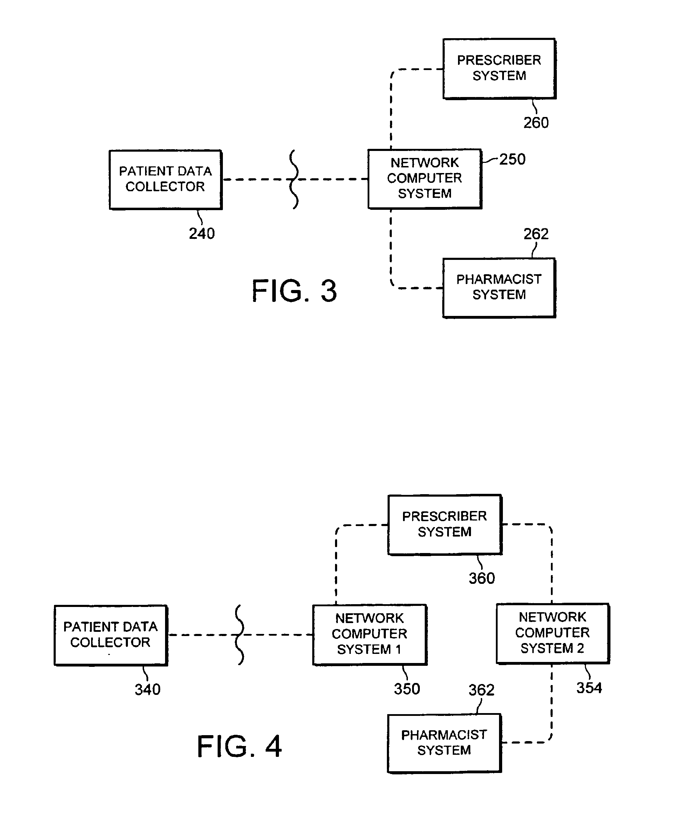

Figure 3 shows a system herein in which patient electronic data collector 240 communicates wirelessly with geographically distant network computer system 250. The network computer system 250 is itself accessible by the system of a medicament prescriber 260 (e.g. a doctor's surgery system) and by the system of a pharmacist 262.

Figures 3 and 4 show representative parts of systems herein in which the patient electronic data collector is shown schematically. It will be appreciated that the full system will comprise plural such patient electronic data collectors. It will also be appreciated that the network computer system will have a particular arrangement and structure as will become apparent when reference is made to later Figures presented herein.

The system of Figure 3 may be employed in the remote assessment of a patient and electronic prescribing therefor as follows. The patient data collector 240 communicates data relating to the medical condition of the patient to the network computer system 250. The medicament prescriber 260 accesses this data e.g. by use of a palmtop communications and data management device and makes a judgement as to prescription needs. If a new prescription is needed the prescriber sends a 'prescription authorisation' signal to the network computer system 250. The pharmacist 262 then accesses the network computer system to receive the 'prescription authorisation' signal which authorises them to make up the prescription for the patient.

The system of Figure 4 is a variation of the system of Figure 3 in which patient electronic data collector 340 communicates wirelessly with geographically distant network computer system 350. The network computer system 350 is itself accessible by the system of a medicament prescriber 360 (e.g. a doctor's surgery system). The prescriber system 360 may also access second network computer system 354 which is accessible by the system of a pharmacist 362. In an alternative herein, the second computer system 354 may be integral with the system of the pharmacist 362 or be a dedicated secure prescription system accessible only to the prescriber and the pharmacist.

The system of Figure 4 is employed in the remote assessment of a patient and electronic prescribing therefor as follows. The patient data collector 340 communicates data relating to the medical condition of the patient to the network computer system 350. The medicament prescriber 360 accesses this data and makes a judgement as to prescription needs. If a new prescription is needed the prescriber sends a 'prescription authorisation' signal to the second network computer system 354. The pharmacist 362 then accesses the network computer system to receive the 'prescription authorisation' signal which authorises the pharmacist to make up the prescription for the patient.

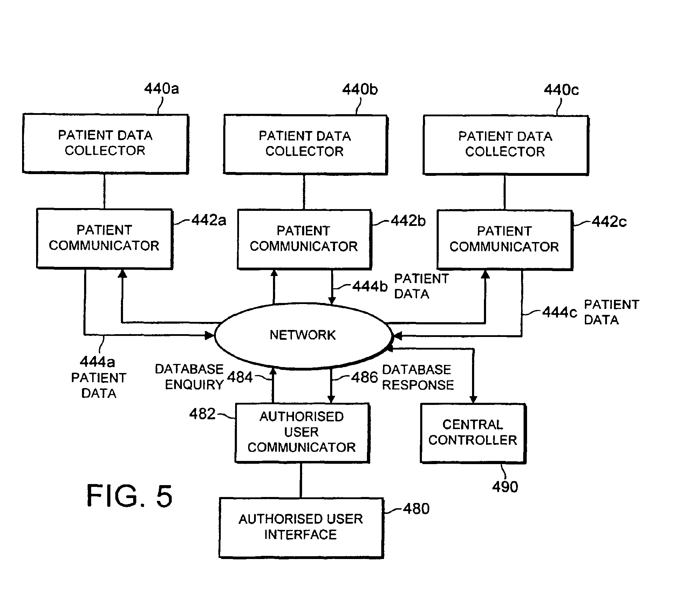

Figure 5. shows a representative system herein comprising plural patient electronic data collectors 440a-c, each of which would be under the control of a different patient. Associated with each patient electronic data collector 440a-c there is a patient communicator 442a-c (e.g. a modem) which is capable of communication with a network computer system 450. The system also comprises an authorised user interface 480 having associated authorised user communicator 482 which is capable of communicating with the network computer system 450. Central controller unit 490 is in two-way communication with the network computer system 450.

The system of Figure 5. is shown in patient 'data upload mode' wherein patient data 444a-c is being communicated to the network. It may be appreciated that any patient can also communicate requests for data to the network 450 and receive responses thereto via the patient communicator 442a-c. The system is also shown in authorised user 'enquiry mode' in which a database enquiry 484 is communicated to the network computer system 450 and a response received 486 via the authorised user communicator 482 to the authorised user interface 480.

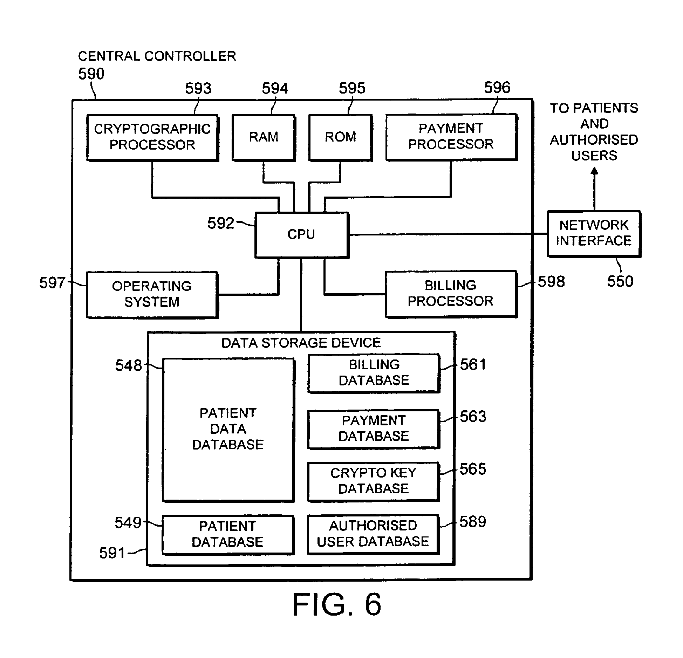

Figure 6. shows the structure of the central controller 590 in more detail. The central controller includes a data storage device 591, central processor (CPU) 592, cryptographic processor 593, RAM 594, ROM 595, payment processor 596, operating system 597 and billing processor 598.

The components of the central controller 590 must be selected to be capable of handling sufficiently large volumes of data. The data storage devices, processors and operating system and other components may be selected from those commercially available.

The data storage device 591 is partitioned to include plural databases. The databases comprise a patient data database 548 which comprises diagnostic or compliance data communicated from any patient electronic data collector; a patient database 549 which includes patient details such as name, address and possibly medical history; an authorised user database 589 which includes details of authorised users of the system; a billing database 561 for billing authorised users on retrieval of data; a payment database 563 for payment of patients or other authorised users in return for data provided; and a crypto key database 565 comprising encryption information.

It will be appreciated that various parts of the system are designed to co-operate with each other in use. For example, the cryptographic processor 593 will access the crypto key database 565 to enable performance of user authentication. Together these crypto elements may form a secure access gateway to the patient data database.

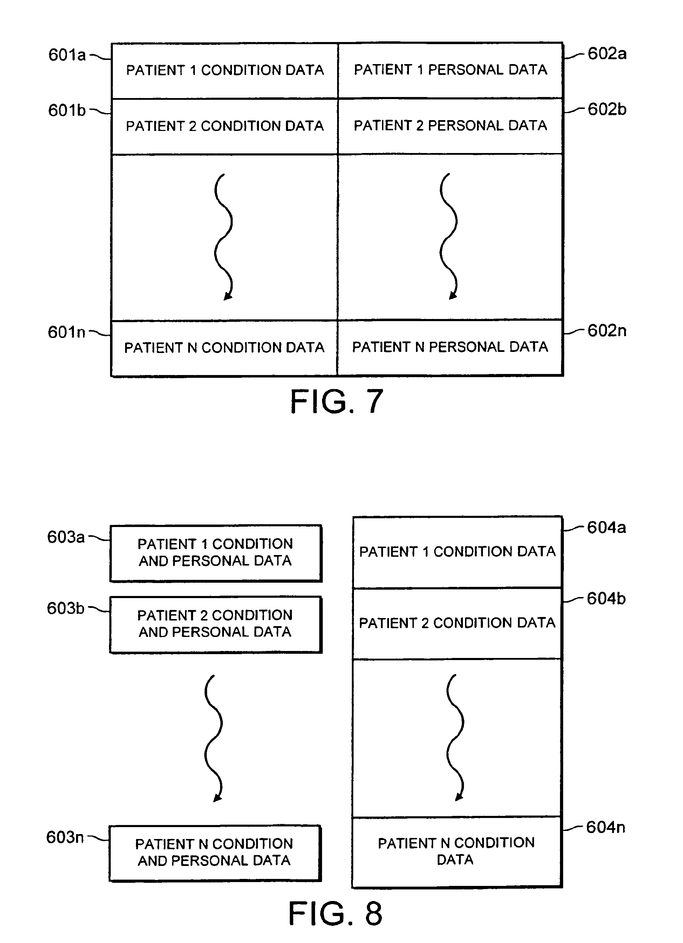

As shown in Figure 6, the patient data database comprises a single database, but the database will usually be either partitioned as shown in Figure 7 or comprise two distinct databases as shown in Figure 8. Access to the patient data will be restricted according to level of access authorisation. In general, the patient and possibly their doctor will be allowed access to patient-specific data and personal details. Other authorised users will be permitted access to non-personal condition-specific data which provides an overview of the medical condition of the patients whose data is comprised therein.

In more detail, Figure 7 shows a single database comprising data relating to plural patients. The database is partitioned into a patient condition database 601 a-n which comprises patient data relating to medical condition which has been collected from each patient, and a patient personal data database 602a-n which comprises patient personal data including medical history data. Different levels of user authorisation may be established to allow different users to access each part of the partitioned database.

Also in more detail, Figure 8 shows a different database structure. The first part of the database structure comprises plural patient condition and personal data databases 601 a-n which comprises patient data relating to medical condition which has been collected from each patient and patient personal data. The second part of the database structure comprises patient data relating to medical condition which has been collected from each patient but with no patient identification or medical history details. Again different levels of user authorisation may be established to allow different users to access each part of the database structure.

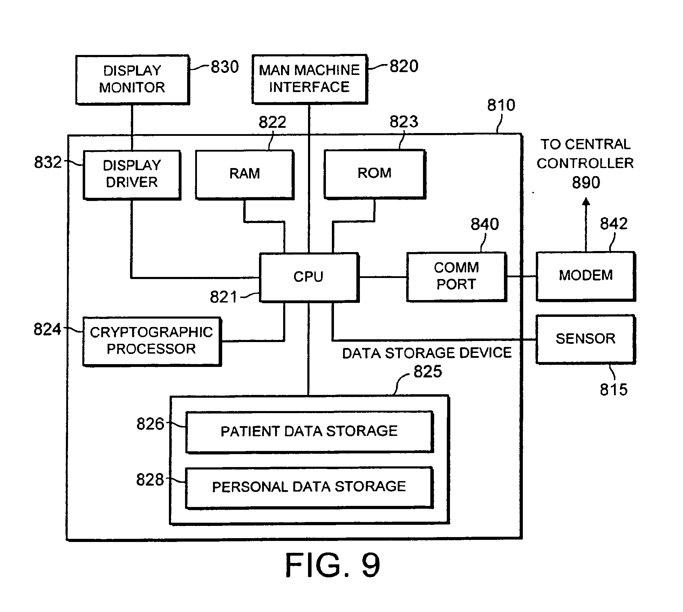

Figure 9 shows a patient electronic data collector 810 comprised with a respiratory drug delivery system (not shown). The electronic data collector 810 comprises a central processor unit (CPU) 821; RAM 822; ROM 823 and a cryptographic processor 824. The CPU 821 receives patient data from sensor 815 which may for example be a breath sensor or a sensor detecting actuation of the drug delivery system. The received data is storable in data storage device 825 which includes two databases, one for storage of patient medical data 826 and one for storage of personal patient data 828. The CPU 821 is associated with man machine interface 820 for receipt of patient input commands and display driver 832 and display 830 for display of information to the patient. The CPU 821 is further associated with communications port 840 which links via modem 842 to the central controller 890 of a network computer system (not shown).

It will be appreciated that the basic structure of the patient electronic data collector 810 of Figure 9 can act as an authorised data communicator for making enquiry requests to the databases on the network computer system and receiving responses therefrom. It will also be appreciated that the structure of the patient electronic data collector could be adapted by removal of the sensor 815 to form a non-patient authorised data communicator which would not be comprised within a drug delivery system.

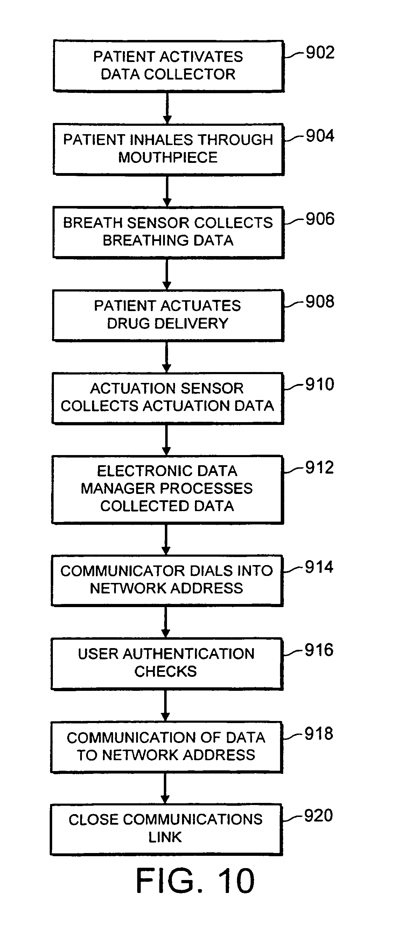

In the flow diagram of Figure 10. the steps involved in a typical data collection and upload procedure are illustrated. Initially a patient activates 902 a data collector system which is located on a respiratory drug delivery system (as in Figure 1). The patient inhales through the mouthpiece 904 and a breath sensor collects data 906 relating to the patient's breath. The patient then actuates the system for drug delivery 908. Actuation may be manually actuated or it may be actuated in response to the patient's inhalation through the mouthpiece 904. An actuation sensor collects actuation data 910, for example relating to the time of actuation and type of drug delivered. The breath and actuation data is collected and processed by an electronic data manager 912. Either automatically, or in response to a request by the patient the communicator dials into the patient network address on the network computer system 912. Before access is permitted user authentication checks 916 are undertaken typically involving a password login. Once access is granted the data is communicated to the network address 918. Once data upload is completed the communications link is closed 920.

In the flow diagram of Figure 10. the steps involved in a typical data search and review procedure are illustrated. Following activation by an authorised user 1022, a communicator dials into the network computer system 1024. Before access is permitted user authentication checks 1026 are undertaken typically involving a password login procedure. A database search menu interface is presented to the authorised user 1028. The choices of database presented by the search menu to the user will depend upon the level of user authorisation. Search parameters are selected 1030 and a search of the patient database undertaken 1032. A check step is undertaken to check if the authorised user is permitted to download search data for offline review 1034. If download is permitted, the results of the search are communicated to the authorised user 1036 for analysis offline. If download of search data is not permitted the system enables online review of the data at the network address 1040. Further searches may be undertaken if desired. Following either data download or online review of the search data the user indicates an intention to exit the system by closing the communications link 1044. Prior to exit of the system an electronic billing request is transmitted to the user 1042. Payment may either occur instantaneously by deduction of funds from the authorised user's account or payment may become due at a later date.

The medicament delivery system aspect of the invention is in one aspect suitable for dispensing medicament for the treatment of respiratory disorders such as disorders of the lungs and bronchial tracts including asthma and chronic obstructive pulmonary disorder (COPD).

Appropriate medicaments may thus be selected from, for example, analgesics, e.g., codeine, dihydromorphine, ergotamine, fentanyl or morphine; anginal preparations, e.g., diltiazem; antiallergics, e.g., cromoglycate (eg s the sodium salt), ketotifen or nedocromil (eg as the sodium salt); antiinfectives e.g., cephalosporins, penicillins, streptomycin, sulphonamides, tetracyclines and pentamidine; antihistamines, e.g., methapyrilene; anti- inflammatories, e.g., beclomethasone (eg as the dipropionate ester), fluticasone (eg as the propionate ester), flunisolide, budesonide, rofleponide, mometasone eg as the furoate ester), ciclesonide, triamcinolone (eg as the acetonide) or 6α, 9α-difluoro-11β-hydroxy-16α-methyl-3-oxo-17α-propionyloxy-androsta-1,4-diene-17β-carbothioic acid S-(2-oxo-tetrahydro-furan-3-yl) ester; antitussives, e.g., noscapine; bronchodilators, e.g., albuterol (eg as free base or sulphate), salmeterol (eg as xinafoate), ephedrine, adrenaline, fenoterol (eg as hydrobromide), formoterol (eg as fumarate), isoprenaline, metaproterenol, phenylephrine, phenylpropanolamine, pirbuterol (eg as acetate), reproterol (eg as hydrochloride), rimiterol, terbutaline (eg as sulphate), isoetharine, tulobuterol or 4-hydroxy-7-[2-[[2-[[3-(2-phenylethoxy)propyl]sulfonyl]ethyl]amino]ethyl-2(3H)-benzothiazolone; adenosine 2a agonists, eg 2R,3R,4S,5R)-2-[6-Amino-2-(1S-hydroxymethyl-2-phenyl-ethylamino)-purin-9-yl]-5-(2-ethyl-2H-tetrazol-5-yl)-tetrahydro-furan-3,4-diol (e.g. as maleate); α₄ integrin inhibitors eg (2S)-3-[4-({[4-(aminocarbonyl)-1-piperidinyl]carbonyl}oxy)phenyl]-2-[((2S)-4-methyl-2-{[2-(2-methylphenoxy) acetyl]amino}pentanoyl)amino] propanoic acid (e.g as free acid or potassium salt), diuretics, e.g., amiloride; anticholinergics, e.g., ipratropium (eg as bromide), tiotropium, atropine or oxitropium; hormones, e.g., cortisone, hydrocortisone or prednisolone; xanthines, e.g., aminophylline, choline theophyllinate, lysine theophyllinate or theophylline; therapeutic proteins and peptides, e.g., insulin or glucagon; vaccines, diagnostics, and gene therapies. It will be clear to a person skilled in the art that, where appropriate, the medicaments may be used in the form of salts, (e.g., as alkali metal or amine salts or as acid addition salts) or as esters (e.g., lower alkyl esters) or as solvates (e.g., hydrates) to optimise the activity and/or stability of the medicament.

Preferred medicaments are selected from albuterol, salmeterol, fluticasone propionate and beclomethasone dipropionate and salts or solvates thereof, e.g., the sulphate of albuterol and the xinafoate of salmeterol.

Medicaments can also be delivered in combinations. Preferred formulations containing combinations of active ingredients contain salbutamol (e.g., as the free base or the sulphate salt) or salmeterol (e.g., as the xinafoate salt) or formoterol (eg as the fumarate salt) in combination with an antiinflammatory steroid such as a beclomethasone ester (e.g., the dipropionate) or a fluticasone ester (e.g., the propionate) or budesonide. A particularly preferred combination is a combination of fluticasone propionate and salmeterol, or a salt thereof (particularly the xinafoate salt). A further combination of particular interest is budesonide and formoterol (e.g. as the fumarate salt).

It will be understood that the present disclosure is for the purpose of illustration only and the invention extends to modifications, variations and improvements thereto.

The application of which this description and claims form part may be used as a basis for priority in respect of any subsequent application. The claims of such subsequent application may be directed to any feature or combination of features described therein. They may take the form of product, method or use claims and may include, by way of example and without limitation, one or more of the following claims:

## Claims

1. A system for collecting and providing selective access to medical data relevant to plural patients having related medical conditions comprising
a network computer system (50; 150; 450);
associated with the network computer system, a first patient-specific database (602a-n; 604a-n) and a second condition-specific database (601a-n; 603a-n)
remote from said network computer system, plural patient electronic data collectors (440a-c), each for collecting patient data relevant to a particular patient's medical condition;
associated with each patient electronic data collector, a communicator (442a-c) for communicating with an entrypoint to said network computer system to enable transfer of said patient data to said first patient-specific database and to said second condition-specific database;
a first secure access gateway (480) permitting access to the first patient-specific database in response to a first user authorisation command; and
a second secure access gateway permitting access to the second condition-specific database in response to a second user authorisation command,
**characterized in that** the first patient-specific database and the second condition-specific database are separate from each other, wherein said first patient-specific database is set up to store the patient data relevant to plural particular patients arranged on a patient-specific basis, and said second medical condition-specific database is set up to store the patient data relevant to plural particular patients arranged on a medical condition-specific basis.

2. A system according to claim 1, wherein said patient electronic data collector collects said patient data on a regular basis.

3. A system according to claim 2, wherein the patient electronic data collector collects the patient data on a continuous basis.

4. A system according to any of claims 1 to 3, wherein said patient data comprises diagnostic data for use in diagnosing each patient's medical condition.

5. A system according to any of claims i to 3, wherein said patient data comprises compliance data for use in assessing each patient's compliance with a treatment or prescribing regime.

6. A system according to any of claims 1 to 5, wherein each patient has a similar or identical medical condition.

7. A system according to any of claims 1 to 6, wherein each patient is on a similar or identical treatment or prescribing regime.

8. A system according to any of claims 1 to 7, wherein each patient electronic data collector is under the control of an individual patient.

9. A system according to any of claims 1 to 8, wherein any patient electronic data collector is integrated with a system for the delivery of medicament.

10. A system according to claim 9, wherein the medicament delivery system provides respirable delivery of medicament to the patient.

11. A system according to claim 9, wherein the medicament delivery system provides injectable delivery of medicament to the patient.

12. A system according to claim 9, wherein the medicament delivery system is an implant in the body of the patient.

13. A system according to any of claim 9 to 11, wherein the patient electronic data collector and the system for delivery of medicament are comprised within a handheld device.

14. A system according to any of claims 1 to 13, wherein the communicator (442a-c) is capable of communicating wirelessly with the entrypoint to the network computer system.

15. A system according to any of claims 1 to 14, wherein the patient data is communicable between the patient electronic data collector and the network computer system in encrypted form.

16. A system according to any of claims 1 to 15, wherein the patient data is continuously communicable between the patient electronic data collector and the network computer system.

17. A system according to any of claims 1 to 16, wherein the patient data is communicable in packet form between the patient electronic data collector and the network computer system.

18. A system according to any of claims 1 to 17, wherein the network computer system is under the control of a healthcare data manager.

19. A system according to claim 18, wherein the healthcare data manager is associated with a healthcare organisation selected from the group consisting of a doctor's practice, a hospital, a healthcare management centre and a pharmaceutical company.

20. A system according to any of claims 1 to 19, wherein the data within either database is partitioned according to level of confidentiality or level of commercial sensitivity.

21. A system according to any of claims 1 to 20, wherein the first and second secure access gateways are distinct from each other.

22. A system according to any of claims 1 to 20, wherein the first and second secure access gateways are coupled or arranged in series.

23. A system according to any of claims 1 to 22, wherein the secure access gateways are password protected.

24. A system according to any of claims 1 to 23, wherein the first and second user authorisation commands are distinct.

25. A system according to any of claims 1 to 23, wherein the first and second user authorisation commands are identical.

26. A system according to any of claims 1 to 25, wherein the authorised users are selected from the group consisting of the patient, a healthcare professional, a pharmacist, an emergency assistance provider, a research professional, a database manager and any combinations thereof.

27. A system according to any of claims 1 to 26, wherein access to either one or both of the databases involves payment of a fee through an electronic payment means.

28. A system according to any of claims 1 to 27, wherein communication of patient data to either one or both of the databases results in award of an incentive payment through an electronic payment means.

29. A system according to any of claims 1 to 28, enabling the patient to define permissions or authorisations at the time of data collection, data transfer, data storage and data access.

30. A system according to any of claims 1 to 29, wherein information from a remote datasource is made available to the network computer system.

31. A system according to claim 30, wherein said remote datasource comprises data relating to ambient environmental conditions.

32. A system according to claim 30, wherein the remote datasource comprises a database of prescribable medicaments.

33. A system according to any of claims 1 to 32, wherein the patient electronic data collector further comprises a patient electronic data management system comprising
a memory for storage of data;
a microprocessor for performing operations on said data; and
a transmitter for transmitting a signal relating to the data or the outcome of an operation on the data.

34. A system according to claim 33, wherein said patient electronic data management system additionally comprises a geographic positioning system.

35. A system according to either of claims 33 or 34, wherein the communicator enables two-way transfer of data between the network computer system and the patient electronic data management system.

36. A system according to any of claims 1 to 35, additionally comprising an authorised user data communicator comprising
an authorised user electronic data management system comprising
a memory for storage of data;
a microprocessor for performing operations on said data; and
a transmitter for transmitting a signal relating to the data or the outcome of an operation on the data; and
a user communicator (482) for communicating with an entrypoint to a network computer system to enable communication of data between the network computer system and the authorised user electronic data management system.

37. A system according to claim 36 for the remote assessment of a patient's medical condition and remote prescription therefor comprising
a first authorised user data communicator capable of communicating a prescription authorisation command to the network computer system; and
a second authorised user data communicator capable of receiving a prescription authorisation command from the network computer system.

38. A system according to any of claims 1 to 37, wherein any communicator employs radiofrequency or optical signals.

39. A system according to any of claims 1 to 38, wherein any communicator communicates with the network computer system via a gateway thereto.

40. A system according to any of claims 1 to 38, wherein the communicator includes an embedded network server.

41. A system according to any of claims 1 to 40, wherein the communicator communicates with the network computer system via a second communications device having telecommunications capability.

42. A system according to claim 41, wherein the telecommunications device comprises a cellular phone or pager.

43. A system according to any of claims 40 to 42, wherein the user communicator (482) communicates with the second communications device using spread spectrum radiofrequency signals.

44. A system according to any of claims 1 to 43, wherein the communicator communicates with a specifiable network address of the network computer system.

45. A system according to claim 44, wherein the specifiable network address is selected from the group consisting of a web-site address, an e-mail address and a file transfer protocol address.

46. A system according to any of claims 33 to 45, wherein the patient electronic data management system additionally comprises a data input system for patient input of data to the electronic data management system.

47. A system according to claim 46, wherein said data input system comprises a man machine interface selected from a keypad, graphical user interface (GUI), voice recognition interface or biometrics interface.

48. A system according to any of claims 33 to 47, additionally comprising a display for display of data from the patient electronic data management system to the patient.

49. A system according to any of claims 1 to 48, wherein the plural patients have related respiratory conditions and each patient electronic data collector additionally comprises a sensor which senses the breath of a user, wherein the sensor communicates breath data to the patient electronic data collector.

50. A system according to claim 49, wherein said sensor comprises a breath-movable element which is movable in response to the breath of a patient.

51. A system according to claim 50, wherein said breath-movable element is selected from the group consisting of a vane, a sail, a piston and an impeller.

52. A system according to claim 49, wherein the sensor comprises a pressure sensor for sensing the pressure profile associated with the breath of a user.

53. A system according to claim 49, wherein the sensor comprises an airflow sensor for sensing the airflow profile associated with the breath of a user.

54. A system according to claim 49, wherein the sensor comprises a temperature sensor for sensing the temperature profile associated with the breath of a user.

55. A system according to claim 49, wherein the sensor comprises a moisture sensor for sensing the moisture profile associated with the breath of a user.

56. A system according to claim 49, wherein the sensor comprises a gas sensor for sensing the oxygen or carbon dioxide profile associated with the breath of a user.

57. A system according to any of claims 49 to 56, wherein said breath data includes breath cycle data.

58. A system according to any of claims 49 to 56, wherein said breath data includes peak flow data.

59. A system according to any of claims 1 to 48, wherein the plural patients have related cardiovascular conditions and each patient electronic data collector additionally comprises a sensor which senses the cardiovascular activity of a patient, wherein the sensor communicates cardiovascular data to the patient electronic data collector.

60. A system according to claim 59, wherein said sensor measures the blood pressure of the patient.

## Patentansprüche

1. System zum Sammeln von medizinischen Daten, die für mehrere Patienten relevant sind, die verwandte medizinische Zustände haben, und zum Bereitstellen eines selektiven Zugriffs auf diese, welches System folgendes aufweist:
ein Netzwerk-Computersystem (50; 150; 450);
verbunden mit dem Netzwerk-Computersystem eine erste patientenspezifische Datenbank (602a-n; 604a-n) und eine zweite zustandsspezifische Datenbank (601a-n; 603a-n);
entfernt vom Netzwerk-Computersystem mehrere elektronische Patientendatensammler (440a-c), die jeweils zum Sammeln von Patientendaten dienen, die für einen bestimmten medizinischen Zustand eines Patienten relevant sind;
verbunden mit jedem elektronischen Patientendatensammler einen Kommunikator (442a-c) zum Kommunizieren mit einer Eintrittsstelle zum Netzwerk-Computersystem, um einen Transfer der Patientendaten zu der ersten patientenspezifischen Datenbank und der zweiten zustandsspezifischen Datenbank zu ermöglichen;
ein erstes Gateway für einen sicheren Zugriff (480), das einen Zugriff auf die erste patientenspezifische Datenbank in Reaktion auf einen ersten Anwenderautorisierungsbefehl zulässt; und
ein zweites Gateway für einen sicheren Zugriff, das einen Zugriff auf die zweite zustandsspezifische Datenbank in Reaktion auf einen zweiten Anwenderautorisierungsbefehl zulässt, und
**dadurch gekennzeichnet, dass** die erste patientenspezifisch Datenbank und die zweite zustandsspezifische Datenbank getrennt voneinander sind, wobei die erste patientenspezifische Datenbank aufgebaut ist, um die Patientendaten, die für mehrere bestimmte Patienten relevant sind, angeordnet auf einer patientenspezifischen Basis zu speichern, und die zweite Datenbank, die spezifisch für einen medizinischen Zustand ist, aufgebaut ist, um die Patientendaten, die relevant für mehrere bestimmte Patienten sind, angeordnet auf einer Basis, die spezifisch für einen medizinischen Zustand ist, zu speichern.

2. System nach Anspruch 1, wobei der elektronische Patientendatensammler die Patientendaten auf einer regelmäßigen Basis sammelt.

3. System nach Anspruch 2, wobei der elektronische Patientendatensammler die Patientendaten auf einer kontinuierlichen Basis sammelt.

4. System nach einem der Ansprüche 1 bis 3, wobei die Patientendaten Diagnosedaten zur Verwendung beim Diagnostizieren eines jeweiligen medizinischen Zustands eines Patienten aufweisen.

5. System nach einem der Ansprüche 1 bis 3, wobei die Patientendaten Befolgungsdaten zur Verwendung beim Bewerten, wie jeder Patient ein Behandlungs- oder Verschreibungssystem befolgt, aufweisen.

6. System nach einem der Ansprüche 1 bis 5, wobei jeder Patient einen ähnlichen oder identischen medizinischen Zustand hat.

7. System nach einem der Ansprüche 1 bis 6, wobei jeder Patient unter einem ähnlichen oder identischen Behandlungs- oder Verschreibungssystem ist.

8. System nach einem der Ansprüche 1 bis 7, wobei jeder elektronische Patientendatensammler unter der Steuerung eines individuellen Patienten ist.

9. System nach einem der Ansprüche 1 bis 8, wobei irgendein elektronischer Patientendatensammler mit einem System für die Abgabe eines Medikaments integriert ist.

10. System nach Anspruch 9, wobei das Medikamentenabgabesystem eine einatembare Abgabe eines Medikaments zum Patienten liefert.

11. System nach Anspruch 9, wobei das Medikamentenabgabesystem eine injizierbare Abgabe eines Medikaments zum Patienten liefet.

12. System nach Anspruch 9, wobei das Medikamentenabgabesystem ein Implantat im Körper des Patienten ist.

13. System nach einem der Ansprüche 9 bis 11, wobei der elektronische Patientendatensammler und das System zur Abgabe eines Medikaments innerhalb einer in der Hand gehaltenen Vorrichtung umfasst sind.

14. System nach einem der Ansprüche 1 bis 13, wobei der Kommunikator (442a-c) drahtlos mit der Eintrittsstelle zum Netzwerk-Computersystem kommunizieren kann.

15. System nach einem der Ansprüche 1 bis 14, wobei die Patientendaten zwischen dem elektronischen Patientendatensammler und dem Netzwerk-Computersystem in verschlüsselter Form kommunizierbar sind.

16. System nach einem der Ansprüche 1 bis 15, wobei die Patientendaten zwischen dem elektronischen Patientendatensammler und dem Netzwerk-Computersystem kontinuierlich kommunizierbar sind.

17. System nach einem der Ansprüche 1 bis 16, wobei die Patientendaten in Paketform zwischen dem elektronischen Patientendatensammler und dem Netzwerk-Computersystem kommunizierbar sind.

18. System nach einem Ansprüche 1 bis 17, wobei das Netzwerk-Computersystem unter der Steuerung eines Gesundheitsvorsorge-Datenmanagers ist.

19. System nach Anspruch 18, wobei der Gesundheitsvorsorge-Datenmanager mit einer Gesundheitsvorsorgeorganisation verbunden ist, die aus der Gruppe ausgewählt ist, die aus einer Arztpraxis, einem Krankenhaus, einem Gesundheitsvorsorge-Managementzentrum und einer pharmazeutischen Firma besteht.

20. System nach einem der Ansprüche 1 bis 19, wobei die Daten innerhalb jeder Datenbank gemäß einer Ebene einer Vertraulichkeit oder einer Ebene einer kommerziellen Empfindlichkeit aufgeteilt sind.

21. System nach einem der Ansprüche 1 bis 20, wobei das erste und das zweite Gateway für einen sicheren Zugriff unterschiedlich voneinander sind.

22. System nach einem der Ansprüche 1 bis 20, wobei das erste und das zweite Gateway für einen sicheren Zugriff gekoppelt oder in Reihe angeordnet sind.

23. System nach einem der Ansprüche 1 bis 22, wobei die Gateways für einen sicheren Zugriff passwortgeschützt sind.

24. System nach einem der Ansprüche 1 bis 23, wobei der erste und der zweite Anwenderautorisierungsbefehl unterschiedlich sind.

25. System nach einem der Ansprüche 1 bis 23, wobei der erste und der zweite Anwenderautorisierungsbefehl identisch sind.

26. System nach einem der Ansprüche 1 bis 25, wobei die autorisierten Anwender aus der Gruppe ausgewählt sind, die aus dem Patienten, einem Professionellen auf dem Gebiet der Gesundheitsvorsorge, einem Pharmazeuten, einem Notdienstlieferer, einem Professionellen auf dem Gebiet der Forschung, einem Datenbankmanager und irgendwelchen Kombinationen davon besteht.

27. System nach einem der Ansprüche 1 bis 26, wobei ein Zugriff auf entweder eine oder beide der Datenbanken eine Bezahlung einer Gebühr über eine elektronische Bezahlungseinrichtung enthält.

28. System nach einem der Ansprüche 1 bis 27, wobei eine Kommunikation von Patientendaten zu entweder einer oder beiden der Datenbanken in einer Belohnung durch eine Bezahlung als Anreiz über eine elektronische Bezahlungseinrichtung resultiert.

29. System nach einem der Ansprüche 1 bis 28, das ermöglicht, dass der Patient Zulassungen oder Autorisierungen zur Zeit einer Datensammlung, eines Datentransfers, einer Datenspeicherung und eines Datenzugriffs definiert.

30. System nach einem der Ansprüche 1 bis 29, wobei Information von einer entfernten Datenquelle dem Netzwerk-Computersystem zur Verfügung gestellt wird.

31. System nach Anspruch 30, wobei die entfernte Datenquelle Daten in Bezug auf Umgebungsbedingungen aufweist.

32. System nach Anspruch 30, wobei die entfernte Datenquelle ein Datenbank von verschreibbaren Medikamenten aufweist.

33. System nach einem der Ansprüche 1 bis 32, wobei der elektronische Patientendatensammler weiterhin ein elektronisches Patientendaten-Managementsystem aufweist, das folgendes aufweist:
einen Speicher zur Speicherung von Daten;
einen Mikroprozessor zum Durchführen von Operationen an den Daten; und
einen Sender zum Senden eines Signals in Bezug auf die Daten oder das Ergebnis einer Operation an den Daten.

34. System nach Anspruch 33, wobei das elektronische Patientendaten-Managementsystem zusätzlich ein geografisches Positionierungssystem aufweist.

35. System nach einem der Ansprüche 33 oder 34, wobei der Kommunikator einen Zweiwegetransfer von Daten zwischen dem Netzwerk-Computersystem und dem elektronischen Patientendaten-Managementsystem ermöglicht.

36. System nach einem der Ansprüche 1 bis 35, das zusätzlich einen Kommunikator für Daten für einen autorisierten Anwender aufweist, der folgendes aufweist:
ein elektronisches Managementsystem für Daten für einen autorisierten Anwender, das folgendes aufweist:
einen Speicher zur Speicherung von Daten;
einen Mikroprozessor zum Durchführen von Operationen an den Daten; und
einen Sender zum Senden eines Signals in Bezug auf die Daten oder das Ergebnis einer Operation an den Daten; und
einen Anwenderkommunikator (482) zum Kommunizieren mit einer Eintrittsstelle zu einem Netzwerk-Computersystem, um eine Kommunikation von Daten zwischen dem Netzwerk-Computersystem und dem elektronischen Managementsystem für Daten für einen autorisierten Anwender zu ermöglichen.

37. System nach Anspruch 36 für die entfernte Bewertung eines medizinischen Zustands eines Patienten und ein entferntes Verschreiben dafür, das folgendes aufweist:
einen ersten Datenkommunikator für einen autorisierten Anwender, der einen Verschreibungsautorisierungsbefehl zum Netzwerk-Computersystem kommunizieren kann;
einen zweiten Datenkommunikator für einen autorisierten Anwender, der einen Verschreibungsautorisierungsbefehl vom Netzwerk-Computersystem empfangen kann.

38. System nach einem der Ansprüche 1 bis 37, wobei irgendein Kommunikator Funkfrequenz- oder optische Signale verwendet.

39. System nach einem der Ansprüche 1 bis 38, wobei irgendein Kommunikator mit dem Netzwerk-Computersystem über ein Gateway dorthin kommuniziert.

40. System nach einem der Ansprüche 1 bis 38, wobei der Kommunikator einen eingebetteten Netzwerkserver enthält.

41. System nach einem der Ansprüche 1 bis 40, wobei der Kommunikator mit dem Netzwerk-Computersystem über eine zweite Kommunikationsvorrichtung mit einer Telekommunikationsfähigkeit kommuniziert.

42. System nach Anspruch 41, wobei die Telekommunikationsvorrichtung ein Mobiltelefon oder einen Pager bzw. ein Funkrufgerät aufweist.

43. System nach einem der Ansprüche 40 bis 42, wobei der Anwenderkommunikator (482) mit der zweiten Kommunikationsvorrichtung unter Verwendung von Spreizspektrums-Funkfrequenzsignalen kommuniziert.

44. System nach einem der Ansprüche 1 bis 43, wobei der Kommunikator mit einer spezifizierbaren Netzwerkadresse des Netzwerk-Computersystems kommuniziert.

45. System nach Anspruch 44, wobei die spezifizierbare Netzwerkadresse aus der Gruppe ausgewählt wird, die aus einer Website-Adresse, einer Email-Adresse und einer Dateientransfer-Protokolladresse besteht.

46. System nach einem der Ansprüche 33 bis 45, wobei das elektronische Patientendaten-Managementsystem zusätzlich ein Dateneingabesystem für eine Patienteneingabe von Daten zum elektronischen Daten-Managementsystem aufweist.

47. System nach Anspruch 46, wobei das Dateneingabesystem eine Mensch/Maschinen-Schnittstelle aufweist, die aus einem Tastenfeld, einer grafischen Anwenderschnittstelle (GUI), einer Spracherkennungsschnittstelle oder einer Biometrik-Schnittstelle ausgewählt ist.

48. System nach einem der Ansprüche 33 bis 47, das zusätzlich eine Anzeige zur Anzeige von Daten von dem elektronischen Patientendaten-Managementsystem zum Patienten aufweist.

49. System nach einem der Ansprüche 1 bis 48, wobei die mehreren Patienten verwandte Einatmungszustände haben und jeder elektronische Patientendatensammler zusätzlich einen Sensor aufweist, der den Atem eines Anwenders erfasst, wobei der Sensor Atmungsdaten zu dem elektronischen Patientendatensammler kommuniziert.

50. System nach Anspruch 49, wobei der Sensor ein Element, das mittels Atmung bewegbar ist, aufweist, das in Reaktion auf den Atem eines Patienten bewegbar ist.

51. System nach Anspruch 50, wobei das Element, das mittels Atmung bewegbar ist, aus der Gruppe ausgewählt ist, die aus einem Ventilator, einem Flügel, einem Kolben und einem Flügelrad besteht.

52. System nach Anspruch 49, wobei der Sensor einen Drucksensor zum Erfassen des mit dem Atem eines Anwenders verbundenen Druckprofils aufweist.

53. System nach Anspruch 49, wobei der Sensor einen Luftstromsensor zum Erfassen des mit dem Atem eines Anwenders verbundenen Luftstromprofils aufweist.

54. System nach Anspruch 49, wobei der Sensor einen Temperatursensor zum Erfassen des mit dem Atem eines Anwenders verbundenen Temperaturprofils aufweist.

55. System nach Anspruch 49, wobei der Sensor einen Feuchtigkeitssensor zum Erfassen des mit dem Atem eines Anwenders verbundenen Feuchtigkeitsprofils aufweist.

56. System nach Anspruch 49, wobei der Sensor einen Gassensor zum Erfassen des mit dem Atem eines Anwenders verbundenen Sauerstoff- oder Kohlenstoffdioxidprofils aufweist.

57. System nach einem der Ansprüche 49 bis 56, wobei die Atemdaten Atmungszyklusdaten enthalten.

58. System nach einem der Ansprüche 49 bis 56, wobei die Atmungsdaten Spitzenstromdaten enthalten.

59. System nach einem der Ansprüche 1 bis 48, wobei die mehreren Patienten verwandte Herzgefäßzustände haben und jeder elektronische Patientendatensammler zusätzlich einen Sensor aufweist, der die Herzgefäßaktivität eines Patienten erfasst, wobei der Sensor Herzgefäßdaten zum elektronischen Patientendatensammler kommuniziert.

60. System nach Anspruch 59, wobei der Sensor den Blutdruck des Patienten misst.

## Revendications

1. Système pour collecter et fournir un accès sélectif aux données médicales concernant plusieurs patients ayant des affections liées comprenant
un système informatique en réseau (50 ; 150 ; 450) ;
associé au système informatique en réseau, une première base de données spécifique du patient (602a-n ; 604a-n) et une seconde base de données spécifique de l'affection (601a-n ; 603a-n)
éloignés dudit système informatique en réseau, plusieurs collecteurs de données électroniques du patient (440a-c), chacun pour collecter les données du patient relatives à une affection du patient particulière ;
associé à chaque collecteur de données électroniques du patient, un communicateur (442a-c) pour communiquer avec un point d'entrée avec ledit système informatique en réseau pour permettre le transfert desdites données du patient à ladite première base de données spécifique du patient et à ladite seconde base de données spécifique de l'affection ;
une première passerelle d'accès sécurisé (480) permettant l'accès à la première base de données spécifique du patient en réponse à une première commande d'autorisation de l'utilisateur ; et
une seconde passerelle d'accès sécurisé permettant l'accès à la seconde base de données spécifique de l'affection en réponse à une seconde commande d'autorisation de l'utilisateur,
**caractérisé en ce que** la première base de données spécifique du patient et la seconde base de données spécifique de l'affection sont séparées l'une de l'autre, dans lequel ladite première base de données spécifique du patient est paramétrée pour stocker les données du patient relatives à plusieurs patients particuliers disposées sur une base spécifique du patient, et ladite seconde base de données spécifique de l'affection médicale est paramétrée pour stocker les données du patient relatives à plusieurs patients particuliers disposées sur une base spécifique de l'affection.

2. Système selon la revendication 1, dans lequel ledit collecteur de données électroniques du patient collecte lesdites données du patient régulièrement.

3. Système selon la revendication 2, dans lequel le collecteur de données électroniques du patient collecte les données du patient en continu.

4. Système selon l'une quelconque des revendications 1 à 3, dans lequel lesdites données du patient comprennent des données diagnostiques à utiliser pour le diagnostic de l'affection de chaque patient.

5. Système selon l'une quelconque des revendications 1 à 3, dans lequel lesdites données du patient comprennent les données d'observance à utiliser pour évaluer l'observance de chaque patient d'un traitement ou d'un régime posologique.

6. Système selon l'une quelconque des revendications 1 à 5, dans lequel chaque patient a une affection similaire ou identique.

7. Système selon l'une quelconque des revendications 1 à 6, dans lequel chaque patient est sous un traitement ou régime posologique similaire ou identique.

8. Système selon l'une quelconque des revendications 1 à 7, dans lequel chaque collecteur de données électroniques du patient est sous le contrôle d'un patient individuel.

9. Système selon l'une quelconque des revendications 1 à 8, dans lequel tout collecteur de données électroniques du patient est intégré à un système pour la délivrance de médicaments.

10. Système selon la revendication 9, dans lequel le système de délivrance de médicament fournit une délivrance respirable de médicament au patient.

11. Système selon la revendication 9, dans lequel le système de délivrance de médicament fournit une délivrance injectable du médicament au patient.

12. Système selon la revendication 9, dans lequel le système de délivrance de médicament est un implant dans le corps du patient.

13. Système selon l'une quelconque des revendications 9 à 11, dans lequel le collecteur de données électroniques du patient et le système pour la délivrance de médicament sont compris dans un terminal mobile de poche.

14. Système selon l'une quelconque des revendications 1 à 13, dans lequel le communicateur (442a-c) est capable de communiquer sans fil avec le point d'entrée du système informatique en réseau.

15. Système selon l'une quelconque des revendications 1 à 14, dans lequel les données du patient sont communicables entre le collecteur de données électroniques du patient et le système informatique en réseau sous forme cryptée.

16. Système selon l'une quelconque des revendications 1 à 15, dans lequel les données du patient sont communicables en continu entre le collecteur de données électroniques du patient et le système informatique en réseau.

17. Système selon l'une quelconque des revendications 1 à 16, dans lequel les données du patient sont communicables sous forme de paquet entre le collecteur de données électroniques du patient et le système informatique en réseau.

18. Système selon l'une quelconque des revendications 1 à 17, dans lequel le système informatique en réseau est sous le contrôle d'un gestionnaire de données de santé.

19. Système selon la revendication 18, dans lequel le gestionnaire des données de santé est associé à une organisation de soins de santé sélectionnée dans le groupe composé d'un cabinet de médecin, un hôpital, un centre de gestion des soins de santé et une société pharmaceutique.

20. Système selon l'une quelconque des revendications 1 à 19, dans lequel les données dans l'un ou l'autre des bases de données sont partitionnées selon le niveau de confidentialité ou le niveau de sensibilité commerciale.

21. Système selon l'une quelconque des revendications 1 à 20, dans lequel les première et seconde passerelles d'accès sécurisés sont distinctes l'une de l'autre.

22. Système selon l'une quelconque des revendications 1 à 20, dans lequel les première et seconde passerelles d'accès sécurisé sont couplées ou disposées en série.

23. Système selon l'une quelconque des revendications 1 à 22, dans lequel les passerelles d'accès sécurisé sont protégées par un mot de passe.

24. Système selon l'une quelconque des revendications 1 à 23, dans lequel les première et seconde commandes d'autorisation de l'utilisateur sont distinctes.

25. Système selon l'une quelconque des revendications 1 à 23, dans lequel les première et seconde commandes d'autorisation de l'utilisateur sont identiques.

26. Système selon l'une quelconque des revendications 1 à 25, dans lequel les utilisateurs autorisés sont sélectionnés dans le groupe composé du patient, d'un professionnel de santé, d'un pharmacien, d'un prestataire de secours d'urgence, d'un professionnel de la recherche, d'un gestionnaire de base de données, et de toute combinaison de ceux-ci.

27. Système selon l'une quelconque des revendications 1 à 26, dans lequel l'accès à l'une des bases de données ou aux deux implique le paiement d'un droit par un moyen de paiement électronique.

28. Système selon l'une quelconque des revendications 1 à 27, dans lequel la communication des données du patient à l'une des bases de données ou les deux entraîne l'attribution d'une prime par un moyen de paiement électronique.

29. Système selon l'une quelconque des revendications 1 à 28, permettant au patient de définir des permissions ou des autorisations au moment de la collecte des données, du transfert des données, du stockage des données et de l'accès aux données.

30. Système selon l'une quelconque des revendications 1 à 29, dans lequel les informations d'une source de données distante sont rendues disponibles pour le système informatique en réseau.

31. Système selon la revendication 30, dans lequel ladite source de données distante comprend des données relatives aux conditions environnementales ambiantes.

32. Système selon la revendication 30, dans lequel la source de données distante comprend une base de données des médicaments pouvant être prescrits.

33. Système selon l'une quelconque des revendications 1 à 32, dans lequel le collecteur de données électroniques du patient comprend en outre un système de gestion des données électroniques du patient comprenant
une mémoire pour le stockage des données ;
un microprocesseur pour réaliser des opérations sur lesdites données ; et
un émetteur pour transmettre un signal relatif aux données ou à la réalisation d'une opération sur les données.

34. Système selon la revendication 33, dans lequel ledit système de gestion des données électroniques du patient comprend en outre un système de positionnement géographique.

35. Système selon l'une quelconque des revendications 33 ou 34, dans lequel le communicateur permet un transfert bidirectionnel des données entre le système informatique en réseau et le système de gestion des données électroniques du patient.

36. Système selon l'une quelconque des revendications 1 à 35, comprenant en outre
un communicateur de données à utilisateur autorisé comprenant
une mémoire pour le stockage des données ;
un microprocesseur pour réaliser des opérations sur lesdites données ; et
un émetteur pour transmettre un signal relatif aux données ou à la réalisation d'une opération sur les données ; et
un communicateur utilisateur (482) pour communiquer avec un point d'entrée avec un système informatique en réseau pour permettre la communication de données entre le système informatique en réseau et le système de gestion des données électroniques de l'utilisateur autorisé.

37. Système selon la revendication 36 pour l'évaluation à distance d'une affection d'un patient et la prescription à distance pour celle-ci comprenant
un premier communicateur de données à utilisateur autorisé capable de communiquer une commande d'autorisation de prescription au système informatique en réseau ; et
un second communicateur de données à utilisateur autorisé capable de recevoir une commande d'autorisation de prescription du système informatique en réseau.

38. Système selon l'une quelconque des revendications 1 à 37, dans lequel tout communicateur emploie des signaux optiques ou de radiofréquence.

39. Système selon l'une quelconque des revendications 1 à 38, dans lequel tout communicateur communique avec le système informatique en réseau par une passerelle à celui-ci.

40. Système selon l'une quelconque des revendications 1 à 38, dans lequel le communicateur comprend un serveur de réseau incorporé.

41. Système selon l'une quelconque des revendications 1 à 40, dans lequel le communicateur communique avec le système informatique en réseau par un second dispositif de communication ayant une capacité de télécommunications.

42. Système selon l'une quelconque des revendications 1 à 41, dans lequel le dispositif de télécommunication comprend un téléphone cellulaire ou un bipeur.

43. Système selon l'une quelconque des revendications 40 à 42, dans lequel le communicateur utilisateur (482) communique avec le second dispositif de communication en utilisant des signaux de radiofréquence à spectre étalé.

44. Système selon l'une quelconque des revendications 1 à 43, dans lequel le communicateur communique avec une adresse réseau pouvant être spécifiée du système informatique en réseau.

45. Système selon la revendication 44, dans lequel l'adresse réseau pouvant être spécifiée est sélectionnée dans le groupe composé d'une adresse d'un site Web, d'une adresse e-mail, et d'une adresse de protocole de transfert de fichiers.

46. Système selon l'une quelconque des revendications 33 à 45, dans lequel le système de gestion des données électroniques du patient comprend en outre un système d'entrée des données pour l'entrée des données par le patient dans le système de gestion des données électroniques.

47. Système selon la revendication 46, dans lequel ledit système d'entrée des données comprend une interface homme machine sélectionnée parmi un clavier, une interface utilisateur graphique (IUG), une interface de reconnaissance vocale ou une interface de biométrie.

48. Système selon l'une quelconque des revendications 33 à 47, comprenant en outre un écran pour l'affichage des données à partir d'un système de gestion des données électroniques du patient pour le patient.

49. Système selon l'une quelconque des revendications 1 à 48, dans lequel les patients multiples ont des affections respiratoires liées et chaque collecteur de données électroniques du patient comprenant en outre un capteur qui détecte la respiration d'un utilisateur, dans lequel le capteur communique les données de la respiration aux collecteurs de données électroniques du patient.

50. Système selon la revendication 49, dans lequel ledit capteur comprend un élément qui peut être déplacé en réponse à la respiration d'un patient.

51. Système selon la revendication 50, dans lequel ledit élément amovible par la respiration est sélectionné dans le groupe composé d'une aube, une aile, un piston et un impulseur.

52. Système selon la revendication 49, dans lequel le capteur comprend un capteur de pression pour capter le profil de pression associé à la respiration d'un utilisateur.

53. Système selon la revendication 49, dans lequel le capteur comprend un détecteur de débit pour détecter le profil de débit associé à la respiration d'un utilisateur.

54. Système selon la revendication 49, dans lequel le capteur comprend un capteur de température pour capter le profil de température associé à la respiration d'un utilisateur.

55. Système selon la revendication 49, dans lequel le capteur comprend un capteur d'humidité pour capter le profil d'humidité associé à la respiration d'un utilisateur.

56. Système selon la revendication 49, dans lequel le capteur comprend un capteur de gaz pour capter le profil d'oxygène ou de dioxyde de carbone associé à la respiration d'un utilisateur.

57. Système selon l'une quelconque des revendications 49 à 56, dans lequel les données de respiration comprennent les données du cycle respiratoire.

58. Système selon l'une quelconque des revendications 49 à 56, dans lequel lesdites données de respirations comprennent les données de débit expiratoire de pointe.

59. Système selon l'une quelconque des revendications 1 à 48, dans lequel les patients multiples ont des affections cardiovasculaires et chaque collecteur de données électroniques du patient comprend en outre un capteur qui capte l'activité cardiovasculaire d'un patient, dans lequel le capteur communique les données cardiovasculaires au collecteur de données électroniques du patient.

60. Système selon la revendication 59, dans lequel ledit capteur mesure la pression artérielle du patient.
